# EUROPEAN PATENT APPLICATION

(11) **EP 2 650 684 A1**
(43) Date of publication of application: **16.10.2013**
(21) Application number: 12163575.9
(22) Date of filing: 10.04.2012
(51) Int. Cl.: G01N 33/68

(54) **Pro SP-B and NT-proBNP based diagnosis in patients with pneumonia**

(71) Applicant: Roche Diagnostics GmbH, 68305 Mannheim (DE); F. Hoffmann-La Roche AG, 4070 Basel (CH)
(72) Inventor: Hess, Georg, 55130 Mainz (DE); Zdunek, Dietmar, 82327 Tutzing (DE); Horsch, Andrea, 68259 Mannheim (DE); Klemt, Volker, 82362 Weilheim (DE); Galluser, Andreas, 82377 Penzberg (DE)
(74) Representative: Dick, Alexander

(57) **Abstract**

The present invention is directed to a method for diagnosing non-cardiac lung damage in a subject suffering from pneumonia, but having no history of heart failure. The method is based on determining the amount of proSP-B in a sample from the subject, and comparing the, thus, determined amount to a reference amount, wherein the reference amount is derived from a reference subject suffering from pneumonia, but having no history of heart failure. The present invention also relates to a method for the early diagnosis of acute lung injury in a subject who suffers from pneumonia. The method is based on the determination of the amount of a brain natriuretic peptide. Further contemplated by the present invention are kits and devices adapted to carry out the methods of the present invention.

## Description

The present invention is directed to a method for diagnosing non-cardiac lung damage in a subject suffering from pneumonia, but having no history of heart failure. The method is based on determining the amount of proSP-B in a sample from the subject, and comparing the, thus, determined amount to a reference amount, wherein the reference amount is derived from a reference subject suffering from pneumonia, but having no history of heart failure. The present invention also relates to a method for the early diagnosis of acute lung injury in a subject who suffers from pneumonia. The method is based on the determination of the amount of a brain natriuretic peptide. Further contemplated by the present invention are kits and devices adapted to carry out the methods of the present invention.

According to the estimates of the WHO, 450 million cases of pneumonia are recorded every year and about four million people die from this illness, accounting for 7 % of fatal mortality of 57 million people (Rudan I. et al Bull Owrld Health Organ 2008: 86: 408 - 16). The majority of these patients suffer from community-aquired pneumonia (CAP). In the US, CAP occurs in approximately 4 million adults and accounts for 10 million physician visits, half a million hospitalisations and 45.000 deaths per year and thus represent the eighth leading cause of death overall in the US (Heron M et al, Natl. Vital Stat Rep 2009: 57: 1- 134). Causes of CAP include viruses (Ruuskanen O. et al Lancet 2011: 377, 1264 - 1275), bacterial and less frequently fungal infections (Navdeep K et al Therapeutic Advances in respiratory Disease 2011: 5: 61 - 78). The risk to develop CAP includes underlying disorders such as active neoplastic disorders, congestive heart failure, cerebrovascular disease, renal disease and liver disease (Navdeep K et al). Mortality from CAP occurs preferentially in elderly patients with multiple comorbidities. In fact, 50 % of deaths in patients with pneumonia are due to comorbidities, such as cardiovascular disease, rather than the initial illness itself (Mortensen E.M. Arch Int Med 2002: 162: 1059 - 1064).

Ware et al. (N Engl J Med 2005;353:2788-96) describes that two fundamentally different types of pulmonary damage occur in humans: cardiogenic pulmonary damage (also termed hydrostatic or hemodynamic edema) and non-cardiogenic pulmonary damage (also known as increased-permeability pulmonary edema, acute lung injury, or acute respiratory distress syndrome). Although they have distinct causes, cardiogenic and non-cardiogenic pulmonary edema may be difficult to distinguish because of their similar clinical manifestations. Knowledge of the cause of acute pulmonary edema has important implications for treatment. Patients with cardiogenic pulmonary edema typically are treated with diuretics and afterload reduction, although the underlying cause may require other treatment, including coronary revascularization. Patients with non-cardiogenic pulmonary edema who require mechanical ventilation should be ventilated with a low tidal volume (6 ml per kilogram of predicted body weight) and a plateau airway pressure less than 30 cm of water. This lung-protective strategy of ventilation reduces mortality in patients with acute lung injury.

According to Ware et al., common causes of cardiogenic pulmonary edema include ischemia with or without myocardial infarction, exacerbation of chronic systolic or diastolic heart failure, and dysfunction of the mitral or aortic valve. Volume overload should also be considered. In contrast, non-cardiogenic pulmonary edema is associated primarily with other clinical disorders, including pneumonia, sepsis, aspiration of gastric contents, and major trauma associated with the administration of multiple blood-product transfusions.

Alveolar damage in patients with pneumonia may occur in asymptomatic patients with and without pre-existing heart failure and, thus, discrimination of non-cardiac lung damage and cardiac lung damage in previously asymptomatic patients may be a challenge. In particular, there is a need of diagnosing non-cardiac lung damage in patients suffering from pneumonia but who have no history of heart failure.

Furthermore, pneumonia may proceed to systemic infection which in turn might present as SIRS or in case of proven or suspected infection to sepsis which in turn might or might not be associated with clinical or subclinical acute lung injury which is associated with alveolar damage. All conditions described, heart failure, pneumonia and acute lung injury might be associated with alveolar damage, thus it is important to dissect these conditions appropriately as this has impact on therapeutic strategies. An important element in this context is NT-proBNP as it increases in heart failure (symptomatic and asymptomatic) but also increases significantly in SIRS/sepsis, see also Kerbaul et al., British Journal of Anaesthesia 93 (5): 639-44 (2004))

In general, the presence of lung damage can be assessed by chest radiography. However, there are several reports that chest radiography may have limited accuracy (see also Ware et al.). The damage may not be visible until the amount of lung water increases by 30 percent. Also, any radiolucent material that fills the air spaces (such as alveolar hemorrhage, pus, and bronchoalveolar carcinoma) will produce a radiographic image similar to that of pulmonary damage. Technical issues can also reduce the sensitivity and specificity of the chest radiograph, including rotation, inspiration, positive-pressure ventilation, position of the patient, and underpenetration or overpenetration of the film. Further, Ware et al. notes that the utility of chest radiography in in distinguishing cardiac from non-cardiogenic lung damage needs further evaluation.

There are four surfactant proteins, the hydrophilic water soluble surfactant proteins A and D (SP-A and SP-D) which are important for host defense, but have less impact than the hydrophobic surfactant proteins on the biophysical, and the hydrophobic surfactant proteins B and C which are critical for optimizing surface tension reduction.

Surfactant Protein B (SP-B) is a 79-amino acid peptide, produced by the proteolytic cleavage of SP-B proprotein (proSP-B) that is processed to a smaller, lipid-associated peptide in the distal secretory pathway within the type II cell. Processing of proSP-B occurs in the multivesicular body (MVB) and lamellar body (LB) compartments.

SP-B facilitates the stability and rapid spreading of surfactant phospholipids during respiratory cycles. It maintains the molecular continuity of the monolayer of the lipid and peptide at the air-water interface during breathing and facilitates the incorporation of lipid from the lung aqueous subphase into the lipid monolayer at the alveolar air-water interface. SP-B is the only surfactant protein absolutely required to initiate breathing and life. Lack of SP-B is invariably lethal shortly after birth. The fully processed mature peptide facilitates organization of surfactant membranes in the lamellar body, likely through its ability to promote membrane-membrane contacts, perturbation of lipid packing, and membrane fusion.

Processing of the SP-B preproprotein to its mature peptide occurs during transit through the secretory pathway from the endoplasmic reticulum to the Golgi network and the multivesicular bodies in the type II epithelial cell.

Entry of the SP-B preproprotein into the secretory pathway is mediated by the N-terminal signal peptide which is cleaved upon translation of the proprotein into the endoplasmic reticulum. Transit of SP-B out of the endoplasmic reticulum is dependent on the N-terminal propeptide, which likely facilitates folding and/or sequestration of the hydrophobic mature peptide. The C-terminal propeptide, (amino acids 280 to 381 of proSP-B) however, seems not to be required for sorting of SP-B to secretory granules.

The technical problem underlying the present invention can be seen as the provision of means and methods for reliably and efficiently diagnosing non-cardiac lung damage in a subject who suffers from pneumonia, but who has no history of heart failure. The technical problem is solved by the embodiments characterized in the claims and herein below.

Accordingly, the present invention relates to a method for diagnosing non-cardiac lung damage in a subject suffering from pneumonia, said subject having no history of heart failure, comprising the steps of
a) determining the amount of proSP-B in a sample from the subject, and
b) comparing the, thus, determined amount to a reference amount,
wherein the reference amount is derived from a reference subject suffering from pneumonia and having no history of heart failure, or from a group thereof.

Preferably, it is diagnosed whether the subject suffers from non-cardiac lung damage, or not, by carrying out the further step of c) diagnosing whether the subject suffers from non-cardiac lung damage, or not, based on the result of the comparison carried out in step b).

The method of the present invention, preferably, is an ex vivo or in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a), (b) and/or (c) may in total or in part be assisted by automation, e.g., by a suitable robotic and sensory equipment for the determination in step (a) or a computer-implemented comparison and/or guidance based on said comparison in step (b).

The phrase "diagnosing non-cardiac lung damage" as used herein, preferably, means assessing whether a subject as referred to herein suffers from non-cardiac lung damage, or not. As will be understood by those skilled in the art, such an assessment is usually not intended to be correct for 100% of the subjects to be diagnosed. The term, however, requires that the assessment is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

In the following, the term "subject" is defined. The definition applies, preferably, to the test subject and the reference subject. The term "subject" as used herein relates to animals, preferably mammals, and, more preferably, humans. Preferably, the subject is an adult. Thus, the subj ect may be 18 years or older.

The subject in accordance with the present invention shall suffer from pneumonia.

The term "pneumonia" is understood by the skilled person. As used herein, the term, preferably, refers to an acute infection of one or both lungs. Pneumonia may be caused by an infection of the lung by bacteria, fungi, protozoa or viruses. In the context of the method of the present invention, pneumonia is preferably caused by bacteria. Bacteria which cause pneumonia include Streptococcus pneumoniae, Staphylococcus aureus, Haemophilus influenzae, Klebsiella pneumoniae, Escherichia coli, Pseudomonas aeruginosa, and Moraxella catarrhalis. Chest pain, dyspnea and fever are other frequently found symptoms. Pneumonia is either primary or secondary pneumonia. Primary pneumonia is, preferably, not precipitated by other pre-existing diseases or disorders. Secondary pneumonia is the complication of another pre-existing disease or disorder. Several diseases increase the risk of pneumonia in a patient. Diseases promoting pneumonia are, preferably, pulmonary edema (caused by heart failure). Pneumonia may also be promoted by a suppressed immune system (e.g., in HIV or cancer patients, in patients suffering from an autoimmune disease, or in patients having a suppressed immune system due treatment with e.g. steroids or anticancer drugs).

The severity of pneumonia can be assessed by determining the pneumonia severity index (PSI). When applying this index, subjects are allocated in five risk class groups, i.e. risk class groups I to V. Whereas patients suffering from risk class IV or V pneumonia have a significantly increased risk of mortality, patients with risk class I, II or III have a low or moderate risk of mortality. How to determine the risk class according to the PSI is described elsewhere herein. In the context of the method of the present invention, the subject shall preferably suffer from risk class I, II, or, more preferably, from risk classes III to V pneumonia. The severity of pneumonia can be also assessed by determining the CURB-65 score which is well known in the art, and which is, e.g., described by Lim et al., 2005, Thorax 58 (5): 377-82. CURB-65, also known as the CURB criteria, is a clinical prediction rule that has been validated for predicting mortality in community-acquired pneumonia. The score is an acronym for each of the risk factors measured. Each risk factor scores one point, for a maximum score of 5: 1. confusion of new onset (defined as an AMT of 8 or less), 2. urea greater than 7 mmol/l (19 mg/dL), 3. respiratory rate of 30 breaths per minute or greater, 4. blood pressure less than 90 mmHg systolic or diastolic blood pressure 60 mmHg or less, 5 age 65 or older. The risk of death at 30 days increases as the score increases. Preferred CURB-65 scores in the context of the present invention are II to V.

Preferably, the pneumonia has been confirmed by chest x ray. Moreover, the pneumonia may be unilateral or bilateral and may involve one or more lobes.

In an embodiment of the present invention, the term "pneumonia" refers to community acquired pneumonia. Thus, the subject to be tested shall not have been recently hospitalized.

Moreover, the subject in the context of the aforementioned method shall have no history of heart failure.

The term "heart failure" is well understood by the skilled person. As used herein, the term an impaired systolic and/or diastolic function of the heart being accompanied by overt signs of heart failure as known to the person skilled in the art.

Systolic and diastolic heart failure can be diagnosed by methods known to the person skilled in the art, preferably echocardiography, in particular tissue Doppler echocardiography (TD). In general, systolic heart failure is apparent by a reduced left ventricular ejection fraction (LVEF). In an embodiment of the present invention, heart failure as used herein is accompanied by a left ventricular ejection fraction (LVEF) of less than 50 % or midwall fractional shortening (MFS) <15%. Diastolic heart failure (DHF) is supposed to account for more than 50% of all heart failure patients and is also referred to as heart failure with normal LVEF ejection fraction (HFNEF). The diagnosis of HFNEF requires the following conditions to be satisfied: (i) signs or symptoms of heart failure; (ii) normal or mildly abnormal systolic LV function; (iii) evidence of diastolic LV dysfunction. Normal or mildly abnormal systolic LV function implies both an LVEF > 50% and an LV end- diastolic volume index (LVEDVI) <97 mL/m2. Diastolic LV dysfunction is preferably diagnosed by tissue Doppler (TD), wherein a ration E/E' > 15 is regarded as diagnostic evidence for diastolic LV dysfunction (E being early mitral valve flow velocity; and E' being early TD lengthening velocity). If TD yields an E/E' ratio suggestive of diastolic LV dysfunction (15 > E/E' > 8), additional non-invasive investigations are required for diagnostic evidence of diastolic LV dysfunction. (e.g. Doppler of the lateral mitral annulus, Doppler of mitral valve or pulmonary veins, echo measures of LV mass index or left atrial volume index, electrocardiographic evidence of atrial fibrillation). For more detailed information on diastolic LV dysfunction, reference is made to the Consensus statement on the diagnosis of heart failure with normal left ventricular ejection fraction by the Heart Failure and Echocardiography Associations of the European Society of Cardiology, European Heart Journal 2007, 28, 2359-2550.

In particular, the test subject and the reference subject shall have no history of heart failure before the onset of symptoms of pneumonia (in particular shortly before the onset of symptoms of pneumonia).

Preferably, the subject having no history of heart failure shall show no symptoms of heart failure (again, preferably before the onset of symptoms of pneumonia). More preferably, the subject is apparently healthy with respect to heart failure. Most preferably, a subject having no history of heart failure is asymptomatic. In particular, the subject is asymptomatic with respect to heart failure.

"A subject who is apparently healthy with respect to heart failure", preferably, has no impaired systolic and diastolic function of the heart. In particular, the subject shall have no impaired systolic and diastolic function of the heart before the onset of symptoms of pneumonia (in particular shortly before the onset of symptoms of pneumonia).

"A subject who is asymptomatic with respect to heart failure" may have impaired heart impaired systolic and/or diastolic function of the heart. However, in accordance with the present invention it is envisaged that the subject who is asymptomatic with respect to heart failure does not show symptoms or overt signs of heart failure. Preferably, the subject can be classified as NYHA class I or NYHA class II patient according to the New York Heart Association (NYHA) classification. Thus, the subject who is asymptomatic for heart failure may show symptoms of NYHA class I or NYHA class II. Symptoms of NYHA class 1 and class II may be not specific for heart failure such as shortness of breath and may occur in diseases other than heart failure. Preferably, the subject is asymptomatic before the onset of symptoms of pneumonia (in particular shortly before the onset of symptoms of pneumonia).

The NYHA classification is well known in the art, see, e.g., "The Criteria Committee of the New York Heart Association. Nomenclature and Criteria for Diagnosis of Diseases of the Heart and Great Vessels". 9th ed. Boston, Mass: Little, Brown & Co; 1994:253-256, which herewith is incorporated by reference in its entirety.

Patients of NYHA Class I have no obvious symptoms of cardiovascular disease but already have objective evidence of functional impairment. Physical activity is not limited, and ordinary physical activity does not cause undue fatigue, palpitation, or dyspnea (shortness of breath).

Patients of NYHA class II have slight limitation of physical activity. They are comfortable at rest, but ordinary physical activity results in fatigue, palpitation, or dyspnea. In contrast, patients of NYHA class III and IV are considered has having a history of heart failure: Patients of NYHA class III show a marked limitation of physical activity. They are comfortable at rest, but less than ordinary activity causes fatigue, palpitation, or dyspnea. Patients of NYHA class IV are unable to carry out any physical activity without discomfort. They show symptoms of cardiac insufficiency at rest.

Preferably, the subject is not a smoker. Furthermore, it is envisaged that the no toxic drugs affecting the lung were administered to the subject such methotrexate and bleomycine. Also, the subject, preferably, shall not have undergone radiation therapy. Moreover, the subject preferably does not suffer from other lung disorders. In particular, the subject does not suffer from COPD (chronic obstructive pulmonary disease), high altitude illness, interstital lung disease, lung cancer, pulmorary aspiration, pulmonary alveolar proteinosis, or from a surfactant deficiency disorder, see e.g. WO 99/13337.

In accordance with the method of the present invention, it shall be diagnosed whether a subject as referred to herein suffers from non-cardiac lung damage, or not. Preferably, the term "lung damage" as used herein refers to damage of pulmonary alveoli, i.e. to alveolar damage. In the lung, the pulmonary alveoli are spherical outcroppings of the respiratory bronchioles and are the primary sites of gas exchange with the blood. Lung damage may have different causes. In particular, lung damage may have cardiac and non-cardiac causes. Cardiac lung damage in accordance with the present invention is lung damage caused by cardiac complications. In particular cardiac lung damage is, preferably, caused by left sided cardiac complications, in particular from left side heart failure, see also Ware et al., *loc. cit.* The term "non-cardiac lung damage" as used herein, preferably, refers to lung damage which is associated with pneumonia, and, thus, preferably caused by pneumonia.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma, urine or serum and, most preferably, blood, plasma or serum. It is particularly preferred to determine the amount of the biomarker in a serum sample. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

The term "proSP-B" relates to a precursor polypeptide of the Surfactant Protein B (SP-B). SP-B is synthesised as a precursor, i.e. proSP-B, and the mature SP-B is obtained by proteolytic cleavage. The mature SP-B is a 79-amino acid hydrophobic peptide that facilitates the stability and rapid spreading of surfactant phospholipids during respiratory cycles. It maintains the molecular continuity of the monolayer of the lipid and peptide at the air-water interface during breathing and facilitates the incorporation of lipids from the lung aqueous subphase into the lipid monolayer at the alveolar air-water interface. Since mature SP-B is comprised in the proSP-B in its N-terminal portion, proSP-B is, preferably, determined by detecting the presence or absence of the C-terminal portion of the proSP-B polypeptide, i.e. C-terminal proSP-B. C-terminal proSP-B in the sense of the present invention relates to proSP-B and all cleavage products or fragments thereof comprising the C-terminal sequence of full length proSP-B. Accordingly, C-terminal proSP-B includes but is not limited to the following: (i) full length proSP-B, i.e. proSP-B comprising the N-terminal propeptide including the amino acid sequence of mature SP-B and the C-terminal proSP-B, (ii) the mid-molecular portion and the C-terminal fragment, i.e. the amino acids from position 201 to 381of proSP-B and (iii) the C-terminal proSP-B fragment, i.e. the amino acids from position 280 to 381 of proSP-B. Amino acid sequences comprising amino acids 1 to 381 for proSP-B are disclosed in Johansson 1992, FEBS Lett. 301:165-167, Jacobs 1987, J Biol Chem 262(20): 9808-11 and Jacobs 1988, J Biol Chem 263(2): 1093, or Pilot-Matias 1989, DNA 8:75-86 and are deposited in UniProtKB/Swiss-Prot data base under accession numbers P07988; Q96R04. proSP-B as used herein encompasses also variants of the aforementioned specific proSP-B polypeptides. Such variants have at least the same essential biological and immunological properties as the specific proSP-B polypeptides. In particular, they share the same essential biological and immunological properties if they are detectable by the same specific assays referred to in this specification, e.g., by ELISA assays using polyclonal or monoclonal antibodies specifically recognizing the said proSP-B polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific proSP-B polypeptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The comparison window, preferably, is the entire length of the query sequence or at least 50% of its length. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith 1981, Add. APL. Math. 2:482, by the homology alignment algorithm of Needleman 1970, J. Mol. Biol. 48:443, by the search for similarity method of Pearson 1988, Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific proSP-B polypeptides or the aforementioned types of variants as long as these fragments have the essential immunological and biological properties as referred to above. Such fragments may be, e.g., degradation products of the proSP-B polypeptides. Further included are variants which differ due to posttranslational modifications such as phosphorylation or myristylation. Moreover, the aforementioned proSP-B may be present as a monomer and/or in dimerized form.

Determining the amount of a peptide or polypeptide referred to in this specification relates to measuring the amount or concentration, preferably, semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay and methods which may utilize labeled molecules in various sandwich, competition, or other assay formats. Such assays are, preferably, based on detection agents such as antibodies which specifically recognize the peptide or polypeptide to be determined. The detection agents shall be either directly or indirectly capable of generating a signal indicating the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immuno-assays (available for example on ElecsysTM analyzers), CBA (an enzymatic Cobalt Binding Assay, available for example on Roche-HitachiTM analyzers), and latex agglutination assays (available for example on Roche-HitachiTM analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Further suitable techniques for the determination of a polypeptide or peptide are described in the following.

First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include 35S, 125I, 32P, 33P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically pro-vides the desired assessment in a suitable output format. The said result may, preferably, serve as an aid for diagnosing non-cardiac lung damage in a patient.

Based on the comparison of the amount determined in step a) and the reference amount, it shall be possible to assess whether the test subject suffers from non-cardiac lung damage, or not. Therefore, the reference amount is to be chosen so that either a difference or an identity in the compared amounts allows identifying those test subjects which belong into the group of subjects which suffer from non-cardiac lung damage, or not. The method allows either excluding (rule-out) or identifying (rule-in) a subject as a subject who suffers from non-cardiac lung damage. Differences in the amounts, i.e. increases or decreases, as used herein, preferably, are differences which are statistically significant. Whether a difference is statistically significant can be determined by the statistical techniques referred to elsewhere herein. Similarly, an identity in the amounts encompasses identical amounts and those differences in the amounts which are not statistically significant and which are within the standard deviations for a measured parameter.

In particular, the method of the present invention allows for identifying a subject who suffers from non-cardiac lung damage, and, thus, to rule in non-cardiac lung damage.

The term "reference amount" as used herein refers to an amount which allows for allocation of a subj ect into either the group of subj ects suffering from non-cardiac lung damage or into the group of subjects not suffering from non-cardiac lung damage. Such a reference amount can be a threshold amount which separates these groups from each other. Accordingly, the reference amount for a the biomarker as referred to herein shall be an amount which allows for allocation of a subject into a group of subjects suffering from non-cardiac lung damage in or into the group of subjects not suffering from non-cardiac lung damage. A suitable threshold amount separating the two groups can be calculated without further ado by the statistical tests referred to herein elsewhere based on amounts of a the biomarker as referred to herein from either a reference subj ect or group of reference subj ects known to suffer from non-cardiac lung damage, or from a reference subject or group of reference subj ects known not to suffer from non-cardiac lung damage.

Reference amounts can, in principle, be calculated for a cohort of subjects as specified above based on the average or mean values for a given biomarker by applying standard methods of statistics. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity versus specificity pairs resulting from continuously varying the decision threshold over the entire range of data observed. The clinical performance of a diagnostic method depends on its accuracy, i.e. its ability to correctly allocate subjects to a certain diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/- specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects suffering from non-cardiac lung damage and subjects not suffering fro, non-cardiac lung damage, preferably, by establishing a ROC for said cohort as described above and deriving a threshold amount therefrom. Dependent on a desired sensitivity and specificity for a diagnostic method, the ROC plot allows deriving suitable thresholds.

Preferably, the reference subject is known not to suffer from non-cardiac lung damage. In this case, an amount of proSP-B in the sample of the test subject which is the same, in particular essentially the same, or which is decreased as compared to the reference amount derived from the sample of the reference subject indicates that the test subject does not suffer from non-cardiac lung damage. A preferred reference amount for proSP-B derived from a sample from a subject known not to suffer from non-cardiac lung damage is within in the range of 25 to 35 ng/ml. Preferably, the reference amount for proSP-B is 35 ng/ml, more preferably, the reference amount is 30 or 25 ng/ml. The aforementioned reference amounts allow, preferably, for ruling out non-cardiac lung damage.

Also preferably, the reference subject is known to suffer from non-cardiac lung damage. In this case, an amount of proSP-B in the sample of the test subject which is the same, in particular essentially the same, or which is increased as compared to the reference amount indicates that the test subject suffers from non-cardiac lung damage. A preferred reference amount for proSP-B derived from a sample from a subject known to suffer from non-cardiac lung damage is within in the range of 35 to 55 ng/ml. Preferably, the reference amount for proSP-B is 35 ng/ml, more preferably, the reference amount is 40 or 45 ng/ml, most preferably, the reference amount is 55 ng/ml The aforementioned reference amounts allow, preferably, for ruling in non-cardiac lung damage.

Corresponding reference amounts for the C-terminal fragment of proSP-B can be determined by the skilled person without further ado. A preferred reference amount for the C-fragment of proSP-B derived from a sample from a subject known to suffer from non-cardiac lung damage is within in the range of 65 to 105 ng/ml. Preferably, the reference amount for proSP-B is 65 ng/ml, more preferably, the reference amount is 80 ng/ml, most preferably, the reference amount is 105 ng/ml The aforementioned reference amounts allow, preferably, for ruling in non-cardiac lung damage. The aforementioned reference amounts allow, preferably, for ruling in non-cardiac lung damage.

Moreover, the reference amount may define a threshold amount, whereby an amount larger than the threshold shall be indicative for a subject suffering from non-cardiac lung damage while an amount lower than the threshold amount shall be an indicator for a subject not suffering from non-cardiac lung damage. The reference amount applicable for an individual subject may vary depending on various physiological parameters such as age, gender, or subpopulation, as well as on the means used for the determination of the polypeptide or peptide referred to herein. A suitable reference amount may be determined by the method of the present invention from a reference sample to be analyzed together, i.e., simultaneously or subsequently, with the test sample. A preferred threshold (i.e., reference amount which is preferably derived from a reference subject referred to herein) for proSP-B is at 35 ng/ml. An amount in the sample of a test subject lower than the reference amount, preferably, indicates that the subject does not suffer from non-cardiac lung damage, whereas an amount in the sample of a test subject larger than the reference amount indicates that the subj ect suffers from cardiac lung damage.

In the course of the studies carried out in the context of the present invention, it has been shown that the reference amount for reliably diagnosing non-cardiac lung damage in a subject suffering from pneumonia, but having no history of heart failure has to be derived from a reference subj ect (or from a group of reference subj ects) suffering from pneumonia, but not having a history of heart failure as well. In particular, it is contemplated that the reference amount is derived from a subject who suffers from pneumonia and who is asymptomatic with respect heart failure. At it can be seen in the Examples section, it was shown that patients who are asymptomatic with respect to heart failure such as patients classified as NYHA class I subject have increased proSP-B levels as compared to healthy subjects even in the absence of pneumonia. The results indicate that these patients - in the absence of pneumonia - exhibit lung damage which is, however, of cardiac origin rather than of pulmonary origin. Thus, increased levels of proSP-B in patients suffering from pneumonia may not necessarily indicate that the patient suffers from lung damage caused by pneumonia. Rather, they may be an indicator of lung damage that was already present at the onset / before the onset of pneumonia. Therefore, in order to reliably diagnosing, in particular ruling in, non-cardiac lung damage, the reference amount shall be derived from a subject who as to be derived from a subject who has no history of heart failure. Preferably, the reference amount shall be derived from a subject being apparently healthy with respect to heart failure (or from a group thereof), if the test subject is also apparently healthy with respect to heart failure. Also preferably, the reference amount shall be derived from a subject being asymptomatic with respect to heart failure (or from a group thereof), if the test subject is also asymptomatic with respect to heart failure. More preferably, the test subject and the reference subject are classified as to belong to the same NYHA class. Thus, if the test subject is classified as a NYHA class I subject, the reference subject shall be classified as NYHA class I subject as well. If the test subject is classified as a NYHA class II subject, the reference subject shall be classified as NYHA class II subject as well.

### Method for diagnosing acute lung injury

The definitions and explanations given herein above apply *mutatis mutandis* to the following, except if stated otherwise.

It has been found in the studies of the present invention that the amount of a brain natriuretic peptide is a reliable marker in a sample of a subject for the early diagnosis of acute lung damage in a subject suffering from pneumonia. The early diagnosis of acute lung damage is important since it detects evidence of early lung damage before development of hyaline membranes, interstitial inflammation and fibrosis and, thus, in a reversible state and before it is detectable by conventional imaging techniques such as x ray.

As set forth above, lung damage can be diagnosed by chest x-ray. However, this method has a limited accuracy. Thus, there is a need for the early diagnosis of acute lung injury in a patient.

Accordingly, the present invention is directed to a method for diagnosing acute lung injury (ALI) in a subject who suffers from pneumonia, said method comprising the steps of
a) determining the amount of a brain natriuretic peptide in a sample from the subject, and
b) comparing the, thus, determined amount to a reference amount,
wherein
i) the reference amount is derived from a reference subject who has no history of heart failure (or from a group thereof), if the subject to be tested has no history of heart failure,
ii) the reference amount is derived from a reference subject who suffers from heart failure classified as NYHA class III (or from a group thereof), if the subject to be tested suffers from heart failure classified as NYHA class III,
iii)the reference amount is derived from a reference subject who suffers from heart failure classified as NYHA class IV (or from a group thereof), if the subject to be tested suffers from heart failure classified as NYHA class IV.

Preferably, it is diagnosed whether the subject suffers from acute lung injury, or not, by carrying out the further step of c) diagnosing whether the subject suffers from acute lung injury, or not, based on the result of the comparison carried out in step b).

The method of the present invention, preferably, is an ex vivo or in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a), (b) and/or (c) may in total or in part be assisted by automation, e.g., by a suitable robotic and sensory equipment for the determination in step (a) or a computer-implemented comparison and/or guidance based on said comparison in step (b).

The phrase "diagnosing acute lung injury" as used herein, preferably, means assessing whether a subject as referred to herein suffers from acute lung injury, or not. As will be understood by those skilled in the art, such an assessment is usually not intended to be correct for 100% of the subjects to be diagnosed. The term, however, requires that the assessment is correct for a statistically significant portion of the subjects (see also comments made elsewhere herein).

The term "subject" has been defined elsewhere herein. The subject in accordance with the aforementioned method shall suffer from pneumonia. Moreover, the subj ect in accordance with the aforementioned method shall be suspected to suffer from SIRS (Systemic inflammatory response syndrome). Furthermore, it is envisaged that the no toxic drugs affecting the lung were administered to the subject such methotrexate and bleomycine. Also, the subject, preferably, shall not have undergone radiation therapy. Moreover, the subject preferably does not suffer from other lung disorders. In particular, the subject does not suffer from COPD (chronic obstructive pulmonary disease), high altitude illness, interstital lung disease, lung cancer, pulmorary aspiration, pulmonary alveolar proteinosis, or from a surfactant deficiency disorder, see e.g. WO 99/13337.

SIRS as used in accordance with the present invention, preferably, encompasses SIRS as defined on the ACCP/SCCM Consensus Conference Definitions (1992/2003) (see e.g. American College of Chest Physicians/Society of Critical Care Medicine Consensus Conference: definitions for sepsis and organ failure and guidelines for the use of innovative therapies in sepsis. Crit Care Med. 1992 Jun;20(6):864-74)). Preferably, a patient is suspected to suffer from SIRS, if the patient displays at least 2 symptoms of the following a) to d), preferably of the following a) to e): a) white blood cell count (WBC) > about 12,000/ µ/L or < about 4000/µ/L, b) body temperature > about 38 0C or < about 36 0C, c) heart rate > about 90 beats/minute, d) a respiratory rate > about 20 breaths/minute or a partial pressure of CO2 of less than about 32 mm Hg, and e) more than 10% immature white blood cells among the counted white blood cells. The white blood cell count is usually determined by automated counting devices. As far as children are concerned, the consensus criteria for diagnosing SIRS in a child are disclosed in Goldstein et al. (Goldstein 2005, Pediatr. Crit Care Med 2005, 6(1),2- 8; see in particular Tables 2 and 3).

Furthermore, in cases where SIRS may develop into sepsis if said disorder is accompanied by a proven or suspected microbial etiology, e.g., a systemic infection. An infection in the sense of the present invention preferably is a viral, fungus or bacterial infection, preferably a bacterial infection associated with bacteria selected from E coli, staphylococcus aureus, Klebsiella pneumoniae, Streptococci or Pseudomonas aeroginosa. The infection may as well be an infection by a fungus selected from Candida albicans, Candida tropicalis or Aspergillus fumigatus. An infection is diagnosed on the basis of assays and criteria generally known to the physician. Preferably, the infection is diagnosed on the basis of a bacterial culture assay, e.g., a culture medium inoculated with a sample from the patient, or based on molecular diagnostic methods. A fungus infection may, for example, be determined based on the generally known test assays such as Septifast. Sepsis may furthermore be determined by the aforementioned criteria for SIRS and at least two of the following criteria in addition: a) presence of leucocytes in a physiologically sterile body fluid; b) peritonitis or perforated viscus; c) pneumonia with focal opacification or petechiae, purpura, or purpura fulminans; d) bacteriemia. Further symptoms or particularly severe complications accompanying SIRS or sepsis are described in standard text books of medicine such as Stadmen or Pschyrembl. Sepsis can proceed to severe sepsis, septic shock, refractory septic shock or multi-organ dysfunction syndrome (MODS). The latter conditions are associated with organ dysfunction, e.g. the cardiovascular, the renal, the respiratory, the cerebral or the hematologic system.

Preferably, the subject to be tested in accordance with the aforementioned method shows an APACHE II Score of 10 or larger. The APACHE II Score includes the assessment of temperature, mean blood pressure, heart rate, respiratory rate, arterial pH, oxygenation, serum sodium, serum potassium, hematocrit and white blood cell count. In addition age, chronic health status and the Glasgow coma Score is considered. Various studies indicate that the APACHE II Score is useful in predicting survival, survival rates have been shown to be higher in postoperative cases at the same APACHE II Score level. The APACHE II scoring system is not restricted to the ICU but can also be performed in the emergency room, this system is however time consuming as it cannot differentiate between different organ failures (Kress J.P., Hall J.B. in Harrison Principles of Internal Medicine p 1673 ff).

In accordance with the aforementioned method, it shall be diagnosed whether a subject who suffers from pneumonia suffers from acute lung injury, or not.

The term "acute lung injury" (ALI) as used herein is well understood by the skilled person (see e.g. Bernard GR, Artigas A, Brigham KL, Carlet J, Falke K, Hudson L et al. The American-European Consensus Conference on ARDS. Definitions, mechanisms, relevant outcomes, and clinical trial coordination. Am J Respir Crit Care Med 1994; 149(3 Pt 1):818-824., here the term "ALI has been clarified). Preferably, the term refers to a critical illness syndrome consisting of acute hypoxemic respiratory failure with bilateral pulmonary infiltrates that are not attributed to left atrial hypertension (See e.g., Rubenfeld GD et al., Incidence and outcomes of acute lung injury, N. Engl. J. Med. 2005, 353:1685-93). In particular, acute lung injury is defined as acute non-cardiac pulmonary lung damage. Thus, the damage shall be acute, i.e. there shall be a sudden onset of the damage. In the context of the aforementioned method, the acute lung injury shall be associated with pneumonia. Thus, it shall be preferably caused by pneumonia.

Acute respiratory distress syndrome (ARDS) is a manifestation of acute injury to the lung. It is the most severe form of acute lung injury that leads to low oxygen levels in the blood and can be life threatening. It has been shown in the context of the studies underlying the present invention that acute lung injury can be diagnosed before the development of ARDS. Therefore, the method of the present invention allows for an early diagnosis of acute lung injury. This is advantageous, since treatment can be initiated before irreversible damage occurs.

The term "brain natriuretic peptide" (BNP) relates to pre-proBNP, proBNP, NT-proBNP, and BNP and variants thereof having the same predictive potential (see e.g. Bonow, 1996, Circulation 93: 1946-1950). Specifically, the aforementioned pre-pro peptide of the brain natriuretic peptide (having 134 amino acids in length) comprises a short signal peptide, which is enzymatically cleaved off to release the pro peptide (108 amino acids). The pro peptide is further cleaved into an N-terminal pro peptide (NT-pro peptide, 76 amino acids) and the active hormone (32 amino acids).

BNP is metabolized in the blood, whereas NT-proBNP circulates in the blood as an intact molecule and as such is eliminated renally. The in-vivo half-life of NTproBNP is 120 min longer than that of BNP, which is 20 min (Smith 2000, J Endocrinol. 167: 239-46.). Prean-alytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20% (Mueller loc.cit.; Wu 2004, Clin Chem 50: 867-73.). Therefore, depending on the time-course or properties of interest, either measurement of the active or the inactive forms of the BNP-type peptides can be advantageous.

The most preferred brain natriuretic peptide referred to herein is human NT-proBNP. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein. The NT-proBNP referred to in accordance with the present invention further encompasses allelic and other variants of said specific sequence for human NT-proBNP discussed above. Specifically, envisaged are variant polypeptides which are on the amino acid level at least 60% identical, more preferably at least 70%, at least 80%, at least 90%, at least 95%, at least 98% or at least 99% identical, to human NT-proBNP. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the polypeptides have NT-proBNP properties. NT-proBNP properties as referred to herein are immunological and/or biological properties. Preferably, the NT-proBNP variants have immunological properties (i.e. epitope composition) comparable to those of NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Invest 59:177-181), Yeo et al. (Yeo 2003, Clinica Chimica Acta 338:107-115). Variants also include posttranslationally modified peptides such as glycosylated peptides. Further, a variant in accordance with the present invention is also a peptide or polypeptide which has been modified after collection of the sample, for example by covalent or non-covalent attachment of a label, particularly a radioactive or fluorescent label, to the peptide.

The term "reference amount" as used herein refers to an amount which allows for allocation of a subject into either the group of subjects suffering from ALI (acute lung injury) or into the group of subjects not suffering from ALI. Such a reference amount can be a threshold amount which separates these groups from each other. Accordingly, the reference amount for a the biomarker as referred to herein shall be an amount which allows for allocation of a subject into a group of subjects suffering from ALI in or into the group of subjects not suffering from ALI. A suitable threshold amount separating the two groups can be calculated without further ado by the statistical tests referred to herein elsewhere based on amounts of a the biomarker as referred to herein from either a reference subject or group of reference subj ects known to suffer from ALI, or from a reference subj ect or group of reference subj ects known not to suffer from ALI. How to calculate a reference amount has been described elsewhere herein.

In the context of the aforementioned method of the present invention, the selection of the reference amount depends on the subject to be tested:

Preferably, the reference amount is derived from a reference subj ect (or to be more precise from a sample of a reference subject) who has not history of heart failure, (or from a group thereof), if the subject to be tested is also has no history of heart failure. In particular, the test subject and the reference subject shall have no history of heart failure before the onset of symptoms of pneumonia (in particular shortly before the onset of symptoms of pneumonia).

For an explanation of the phrase "a subject who has no history of heart failure" see elsewhere herein. Preferably, the subject who has no history of heart failure is selected from the group consisting of a subj ect who is apparently healthy with respect to heart failure and a subject who is asymptomatic with respect to heart failure (for a definition of these terms, please see elsewhere herein). In a preferred embodiment, the subject who is asymptomatic with respect to heart failure has impaired systolic and/or diastolic function of the heart. Accordingly, the subject who is asymptomatic with respect to heart failure may be classified as NYHA class I or II subject. More preferably, the reference amount is derived from a reference subject (or to be more precise from a sample of a reference subject) who is apparently healthy with respect to heart failure, (or from a group thereof), if the subject to be tested is also apparently healthy with respect to heart failure. Also preferably, the reference amount is derived from a reference subject (or to be more precise from a sample of a reference subject) who is asymptomatic with respect to heart failure (or from a group thereof), if the subject to be tested is also asymptomatic with respect to heart failure.

Preferably, the reference amount is derived from a reference subject who suffers from heart failure classified as NYHA class III (or from a group thereof), if the subject to be tested suffers from heart failure classified as NYHA class III. Patients of NYHA class III show a marked limitation of physical activity. In particular, the test subj ect and the reference subject shall suffer from heart failure classified as NYHA class III before the onset of symptoms of pneumonia (in particular shortly before the on-set of symptoms of pneumonia).

Preferably, the reference amount is derived from a reference subject who suffers from heart failure classified as NYHA class IV (or from a group thereof), if the subject to be tested suffers from heart failure classified as NYHA class IV. Patients of NYHA class IV are unable to carry out any physical activity without discomfort. They show symptoms of cardiac insufficiency at rest. In particular, the test subject and the reference subject shall suffer from heart failure classified as NYHA class IV before the onset of symptoms of pneumonia (in particular shortly before the onset of symptoms of pneumonia).

In a preferred embodiment, the reference subject further is known to suffer from acute lung injury. In this case, an amount of the brain natriuretic peptide, in particular of NT-proBNP, in the sample of the test subject which is the same, in particular essentially the same, or which is increased as compared to the reference amount derived from reference subject indicates that the test subject suffers from acute lung injury. In this case the following applies:

A preferred reference amount for NT-proBNP derived from a reference subject who is asymptomatic with respect to heart failure is 500 pg/ml. A preferred reference amount for NT-proBNP derived from a reference subject who suffers from heart failure classified as NYHA class III is 1500 pg/ml. A preferred reference amount for NT-proBNP derived from a reference subject who suffers from heart failure classified as NYHA class III is 5000 pg/ml.

Also preferably, the reference subject is known not to suffer from acute lung injury. In this case, an amount the brain natriuretic peptide, in particular of NT-proBNP, in the sample of the test subject which is the same, in particular essentially the same, or which is decreased as compared to the reference amount derived from the reference subject indicates that the test subject does not suffer from acute lung injury.

Of course, the reference subject shall suffer from pneumonia.

Moreover, the reference amount may define a threshold amount, whereby an amount larger than the threshold shall be indicative for a subject suffering from ALI while an amount lower than the threshold amount shall be an indicator for a subject not suffering from ALI. The reference amount applicable for an individual subject may vary depending on various physiological parameters such as age, gender, or subpopulation, as well as on the means used for the determination of the polypeptide or peptide referred to herein. A suitable reference amount may be determined by the method of the present invention from a reference sample to be analyzed together, i.e., simultaneously or subsequently, with the test sample.

In a preferred embodiment of the aforementioned method, the method further comprises the step of determining the amount of proSP-B in a sample from a subject and comparing the, thus, determined amount to a reference amount. Preferably, the reference is derived from a reference subject as disclosed herein above regarding the reference amount for the brain natriuretic peptide (or from a group thereof).

The following applies as diagnostic algorithm:

Preferably, the reference subject further is known to suffer from acute lung injury. In this case, an amount of the brain natriuretic peptide, in particular of NT-proBNP in the sample of the test subject, and an amount of the brain natriuretic peptide, in particular of NT-proBNP, in the sample of the test subject which are the same, in particular essentially the same, and/or which are increased as compared to the reference amount derived from the reference subject indicates that the test subject suffers from acute lung injury.

Also preferably, the reference subject is known not to suffer from acute lung injury. In this case, an amount the brain natriuretic peptide, in particular of NT-proBNP, in the sample of the test subject, and an amount of the brain natriuretic peptide, in particular of NT-proBNP in the sample of the test subject, which are the same, in particular essentially the same, and/or which are decreased as compared to the reference amount derived from the reference subject indicates that the test subject does not suffer from acute lung injury.

Further, the present invention contemplates the use of the biomarker proSP-B or a detection agent that specifically binds thereto in a sample of a subject suffering from pneumonia, said subject having no history of heart failure, for diagnosing non-cardiac lung damage. The reference amount for carrying out this diagnosis shall be derived from a reference subject (or to be more precise from a sample from a reference subject) suffering from pneumonia, but having no history of heart failure.

Further, the present invention relate to the use of a brain natriuretic peptide, in particular of NT-proBNP, or a detection agent that specifically binds thereto in a sample of a subject suffering from pneumonia for diagnosing acute lung injury. Moreover, included is also the use of a combination of proSP-B and a brain natriuretic peptide or a combination of detection agents therefor for diagnosing acute lung injury. The reference amount (reference amounts) for carrying out this diagnosis shall be derived from a reference subject (reference subjects) as disclosed herein above in the context of the method for diagnosing acute lung injury.

The term "detection agent" as used herein refers to an agent that is capable of specifically recognizing and binding to the biomarker polypeptide(s) present in a sample. Moreover, the said agent shall allow for direct or indirect detection of the complex formed by the said agent and the biomarker. Direct detection can be achieved by including into the agent a detectable label. Indirect labelling may be achieved by a further agent that specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker. Antibodies as referred to herein include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. Preferred antibodies which specifically bind to proSP-B, to the C-fragment of proSP-B, or to NT-proBNP are disclosed in the Examples section.

The present invention also relates to a device adapted for carrying out the method of the present invention for diagnosing non-cardiac lung damage comprising
a) an analyzing unit comprising a detection agent which specifically binds to proSP-B adapted for determining the amount of proSP-B in a sample of a subject suffering from pneumonia who has no history of heart failure; and
b) an evaluation unit for comparing the determined amount with a reference whereby it can be diagnosed whether the subject suffers from non cardiac lung damage, said unit comprising a database with a reference amount from a reference subject suffering from pneumonia who has no history of heart failure and a computer-implemented algorithm for carrying out a comparison as specified herein above.

The present invention also relates to a device adapted for carrying out the method of the present invention for diagnosing acute lung injury comprising
a) an analyzing unit comprising a detection agent which specifically binds to a brain natriuretic peptide, in particular to NT-proBNP, adapted for determining the amount of said brain natriuretic peptide in a sample of a subj ect suffering from pneumonia; and
b) an evaluation unit for comparing the determined amount with a reference whereby it can be diagnosed whether the subj ect suffers from acute lung injury, said unit comprising a database with a reference amount (reference amount) from a reference subject (reference subjects) as set forth in the context of the method for diagnosing acute lung injury and a computer-implemented algorithm for carrying out a comparison as set forth in the context of this method.

In a preferred embodiment of the aforementioned device of the invention, said device further comprises a detection agent for the biomarker proSP-B.

The term "device" as used herein relates to a system comprising the aforementioned units operatively linked to each other as to allow the diagnosis or monitoring according to the methods of the invention. Preferred detection agents which can be used for the analyzing unit are disclosed elsewhere herein. The analyzing unit, preferably, comprises said detection agents in immobilized form on a solid support which is to be contacted to the sample comprising the biomarkers the amount of which is to be determined. Moreover, the analyzing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. The results may be given as output of parametric diagnostic raw data, preferably, as absolute or relative amounts. It is to be understood that these data will need interpretation by the clinician. However, also envisage are expert system devices wherein the output comprises processed diagnostic raw data the interpretation of which does not require a specialized clinician.

The present invention also relates to a kit adapted for carrying out the method of the present invention for diagnosing non-cardiac lung damage comprising a detection agent for the biomarker proSP-B, at least one standard for a reference and instructions for carrying out the said method.

The present invention also relates to a kit adapted for carrying out the method of the present invention for diagnosing acute lung injury comprising a detection agent for the biomarker brain natriuretic peptide, in particular for NT-proBNP, at least one standard for a reference and instructions for carrying out the said method. In a preferred embodiment of the kit of the invention, said kit further comprises a detection agent for the biomarker proSP-B.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as an optical storage medium (e.g., a Compact Disc) or directly on a computer or data processing device. Further, the kit shall comprise at least one standard for a reference as defined herein above, i.e. a solution with a pre-defined amount for the proSP-B or the brain natriuretic polypeptide representing a reference amount. Such a standard may represent, e.g., the amount of proSP-B or of the brain natriuretic peptide from a reference subject or group of reference subjects as disclosed in the context of the methods of the present invention.

All references cited in this specification are herewith incorporated by reference with respect to their entire disclosure content and the disclosure content specifically mentioned in this specification.

### EXAMPLES

### Example 1: Determination of biomarkers NT-proBNP and proSP-B

NT-proBNP was determined with sandwich immuno-assays using COBAS-analyzers from Roche/Hitachi. The assays comprise two monoclonal antibodies specific for the respective peptide. The first of these iv biotinylated and the second one in labelled with a Tris(2,2'-bibyridyl)ruthemium (II)-complex. In a first incubation step both antibodies are incubated with the sample. A sandwich complex comprising the peptide to be determined and the two different antibodies is formed. In a next incubation step streptavidin-coated beads are added to this complex. The beads bind the sandwich complexes. The reaction mixture is then aspirated into a measuring cell where the beads are magnetically captured on the surface of the electrode. The application of a voltage then induces a chemiluminescent emission from the ruthenium complex which is measured by a photomultiplier. The emitted amount of light is dependent on the amount of sandwich complexes on the electrode. NT-proBNP amounts between 2 pg/ml and 35000 pg/ml can be measured.

ProSP-B was tested using newly developed proSP-B tests aiming at the detection of proSP-B and the terminal C fragment of proSP-B (C fragment proSP-B).

The proSP-B assay uses a mouse monoclonal anti-proSP-B (N-terminus) antibody as a capture and a mouse monoclonal anti-proSP-B (C-terminus) antibody as a detection reagent. The assay principle is a sandwich format. The antibody to the N-terminal pro-sequence binds to an epitope comprised in the peptide sequence ranging from amino acid 160 to 169 of proSP-B. The antibody to the C-terminal pro-sequence binds to an epitope comprised in the peptide sequence ranging from amino acid 323 to 334 of proSP-B. Detection is based on an electrochemiluminescence immunoassay (ECLIA), using a Tris(bipyridyl)-ruthenium(II) complex as label. For carrying out the assay, the biotinylated capture antibody (80 µl), the ruthenium-labeled detection antibody (80 µl), and sample or standard material (10 µl) are incubated in homogeneous phase for 9 min at 37°C. Concentrations in the stock solution were 1.7 µg/ml for the biotinylated capture antibody and 1.2 µg/ml for the ruthenylated detection antibody, respectively. After the first nine minutes 30 µl of Streptavidin-coated beads are added, and binding of the immune complexes formed to the microparticles takes place during a second 9-min incubation. After the second incubation, the reaction mixture is transferred into the measuring cell, where beads are captured to the electrode surface by a magnet. The measuring cell is washed to remove unbound label and filled with detection buffer containing Tris-propylamine. After applying voltage to the electrode, the emitted chemiluminescence light is detected by a photo-multiplier. Results are determined via a 2-point calibration curve. The corresponding concentration for proSP-B is given in pg/ml.

The C-fragment proSP-B assay uses a first mouse monoclonal anti-proSP-B (C-terminus) antibody as a capture and a second mouse monoclonal anti proSP-B (C-terminus) antibody as a detection reagent. The assay principle is a sandwich format. The antibody to the first C-terminal pro-sequence binds to an epitope comprised in the peptide sequence ranging from amino acid 323 to 334 of proSP-B. The antibody to the second C-terminal pro-sequence binds to an epitope comprised in the peptide sequence ranging from amino acid 285 to 294 of proSP-B. Detection is based on an electrochemiluminescence immunoassay (ECLIA), using a Tris(bipyridyl)-ruthenium(II) complex as label. For carrying out the assay, the biotinylated capture antibody (MAB 1.7.41) (80 µl), the ruthenium-labeled detection antibody (MAB 1.3.9) (80 µl), and sample or standard material (10 µl) are incubated in homogeneous phase for 9 min at 37°C. Concentrations in the stock solution were 1.5 µg/ml for the biotinylated capture antibody and 1.0 µg/ml for the ruthenylated detection antibody, respectively. After the first nine minutes 30 µl of Streptavidin-coated beads are added, and binding of the immune complexes formed to the microparticles takes place during a second 9-min incubation. After the second incubation, the reaction mixture is transferred into the measuring cell, where beads are captured to the electrode surface by a magnet. The measuring cell is washed to remove unbound label and filled with detection buffer containing Tris-propylamine. After applying voltage to the elec-trode, the emitted chemiluminescence light is detected by a photomultiplier. Results are determined via a 2-point calibration curve. The corresponding concentration for C-terminal proSP-B is given in pg/ml.

### Example 2: Patient cohorts

### a) Patients with Coronary Artery Disease w/o evidence of heart failure:

A total of 158 patients with coronary artery disease who had no concommitant lung disorder and who had no evidence for symptomatic heart failure were also included into the study, these patients had also a normal kidney function as assessed by creatinine values within normal.

### b) Patients with Coronary Artery Disease with evidence of cardiac dysfunction:

A total of 474 patients with stable coronary artery disease and various extent of cardiac dysfunction or heart failure were included into the study. All patients had normal kidney function. In addition they had left ventricular ejection fraction determined by echocardiography. Symptoms of heart failure were recorded according to the NYHA classification. None of the patients had evidence of infection or even pneumonia at the time of presentation and at least 4 weeks before and body temperature was below 37.5 degrees Celsius in all patients.

### c) Patients with Pneumonia:

There were 96 patients with chest X ray proven pneumonia, all patients had normal kidney function at presentation.

### d) Patients with SIRS

In addition 142 patients presenting with SIRS and suspected systemic infection were included into the study. They were also graded according to the APACHE II score according to the suspected severity of SIRS.

### Example 3: Results

### a) Patients with CAD but without concomitant lung disorder und without symptoms specific for heart failure (e.g. NYHA I and II patients) had the following results (separated in 2 groups below (group 1)and above (group 2) median of NT-pro BNP (median NT-pro BNP 116 pg/ml)

**Table:**

| | NT-proBNP | hsTnT | proSP-B | C-fragment |
|---|---|---|---|---|
| | (pg/ml) | (pg/ml) | (ng/ml) | (ng/ml) |
| Group 1 | 54 | 1 | 25 | 50 |
| N=79 | (38 - 83) | (0 - 7) | (20 - 40) | (34 - 79) |
| Group 2 | 227 | 3 | 35 | 65 |
| N=79 | (153 - 292) | (0 - 9) | (26 - 55) | (45 - 105) |

The data obtained indicate that in asymptomatic patients proSP-B below 55 ng/ml and exceeding about 40 ng/ml and C- fragment proSP-B values of below about 105 ng/ml und and exceeding about 79 ng/ml are indicative of cardiac lung damage in asymptomatic patients. ProSP-B values increase with cardiac dysfunction as assessed by NT-proBNP.

### b) Patients with stable CAD and cardiac dysfunction:

NT-pro BNP values of the 474 patients with coronary artery disease and various extent of heart failure are given in the following Table:

| | LVEF | below 30% | 30 - 50% | above 50% |
|---|---|---|---|---|
| | | 27 | 86 | 361 |
| NYHA | N | 2 | 27 | 280 |
| I | NT-proBNP | 2848 | 506 | 302 |
| | (pg/ml) | | | |
| II | N | 6 | 36 | 70 |
| | NT-proBNP | 1896 | 862 | 488 |
| III | N | 16 | 23 | 11 |
| | NT-proBNP | 2467 | 1946 | 698 |
| IV | N | 3 | 0 | 0 |
| | NT-proBNP | 16223 | ND | ND |

The table illustrates that patients with asymptomatic cardiac dysfunction have NT-proBNP values below 500 pg/ml except those who had impaired left ventricular ejection fraction, they had higher NT-pro BNP values even when they were asymptomatic. Symptomatic patients had NT-pro BNP values above 500 pg/ml and in the majority had NT-pro BNP values above 1500 pg/ml.

Patients with systemic infection and meeting the criteria of SIRS were separated into quartiles using the APACHE II score, this is given in the Table below:

| Quartile | APACHE II Score | NT-pro BNP Pg/ml | Death |
|---|---|---|---|
| 1 | 7 (3-9) | 146 (55 -264) | 0 |
| 2 | 13 (11-13) | 841 (258 - 1372) | 1 |
| 3 | 17 (16 - 19) | 1678 (551 - 4599) | 3 |
| 4 | 26 (25 - 31) | 7793 (2342 - 21963) | 6 |

This Table indicates that increasing APACHE II scores are associated with increasing NT-pro BNP levels as a sign of cardiac involvement and with an increased risk of death. When the information of patients with heart failure and those with systemic infection are combined the data indicate that NT-pro value exceeding those in heart failure patients without systemic infection can be attributed to systemic infection. For example a patient who was asymptomatic before he developed pneumonia could have NT-pro BNP values up to 500 pg/ml, if the value measured was found to be below 500 pg/ml, effects of systemic infection appeared unlikely, however if NT-pro BNP values of 1000 pg/ml are found they are considered to be the result of systemic infection (and possible ARDS). Similar consideration apply for patients who had symptomatic NYHA III and IV before they developed pneumonia.

Patients with pneumonia were classified as follows: patients with normal NT-pro BNP values (below 125 pg/ml), patients with NT-pro BNP values consistent with asymptomatic patients with chronic artery disease (125 - 500 pg/ml), patients with NT-pro BNP values consistent with symptomatic heart failure (500 -1500 pg/ml) and patients above 1500 pg/ml. This is presented in the following tables:

### Group 1: NT-proBNP < 125 pg/ml

| | Labor | | | NYHA | Heart failure |
|---|---|---|---|---|---|
| Pat.Id. | proSP-B | C-frag. proSP-B | proBNP | class | |
| | ng/ml | ng/ml | pg/ml | | |
| II-097/1 | 128,43 | 191,49 | 15,00 | n/a | no history |
| II-064/1 | 24,58 | 37,64 | 18,03 | n/a | no history |
| II-284/1 | 118,78 | 180,95 | 59,28 | n/a | no history |
| II-113/1 | 19,00 | 40,04 | 60,82 | n/a | no history |
| II-290/1 | 55,36 | 106,39 | 63,20 | n/a | no history |
| II-280/1 | 70,87 | 209,58 | 65,51 | n/a | no history |
| II-292/1 | 59,65 | 81,04 | 71,41 | n/a | no history |
| II-241/1 | 76,28 | 496,37 | 72,15 | n/a | no history |
| II-182/1 | 36,62 | 68,07 | 80,80 | n/a | no history |
| II-092/1 | 202,71 | 610,48 | 92,11 | n/a | no history |
| II-162/1 | 175,26 | 423,13 | 107,58 | n/a | no history |
| II-059/1 | 33,07 | 50,47 | 122,05 | III | yes |

### Group 2: NT-proBNP 125 - 500 pg/ml

| | Labor | | | NYHA | Heart failure |
|---|---|---|---|---|---|
| Pat.Id. | proSP-B | C-frag. proSP-B | proBNP | class | |
| | ng/ml | ng/ml | pg/ml | | |
| II-184/1 | 598,08 | 1389,95 | 148,11 | n/a | no history |
| II-244/1 | 110,47 | 225,95 | 150,34 | n/a | no history |
| II-163/1 | 59,93 | 137,32 | 150,94 | n/a | no history |
| II-069/1 | 100,45 | 353,80 | 163,77 | III | yes |
| II-079/1 | 779,03 | 1259,42 | 177,40 | II | no history |
| II-278/1 | 476,97 | 2620,74 | 211,64 | n/a | no history |
| II-288/1 | 294,04 | 564,70 | 212,46 | n/a | no history |
| II-061/1 | 56,98 | 120,44 | 250,03 | n/a | yes |
| II-209/1 | 96,62 | 172,46 | 256,39 | n/a | no history |
| II-208/1 | 79,36 | 205,96 | 268,68 | n/a | no history |
| II-011/1 | 173,21 | 326,77 | 284,38 | n/a | no history |
| II-177/1 | 239,58 | 912,53 | 300,43 | II | yes |
| II-105/1 | 32,08 | 53,25 | 319,43 | n/a | no history |
| II-232/1 | 145,61 | 337,01 | 332,60 | n/a | no history |
| II-285/1 | 174,26 | 395,90 | 368,70 | II | yes |
| II-101/1 | 30,75 | 44,27 | 459,87 | n/a | no history |
| II-015/1 | 292,68 | 1152,85 | 491,14 | n/a | no history |

### Group 3: NT-proBNP 500 - 1500 pg/ml

| | Labor | | | NYHA | Heart failure |
|---|---|---|---|---|---|
| Pat.Id. | proSP-B | C-frag. proSP-B | proBNP | class | |
| | ng/ml | ng/ml | pg/ml | | |
| II-275/1 | 54,37 | 105,35 | 519,82 | n/a | no history |
| II-276/1 | 154,46 | 292,53 | 557,56 | n/a | no history |
| II-014/1 | 34,83 | 70,93 | 607,11 | n/a | no history |
| I-07/1 | 78,53 | 187,56 | 621,13 | n/a | yes |
| II-117/1 | 145,99 | 416,85 | 682,30 | n/a | no history |
| II-067/1 | 159,29 | 289,97 | 687,72 | n/a | no history |
| II-289/1 | 360,72 | 627,22 | 697,94 | n/a | no history |
| II-277/1 | 149,11 | 411,29 | 749,10 | n/a | no history |
| II-134/1 | 106,05 | 281,65 | 757,58 | III | yes |
| I-51/1 | 42,50 | 69,95 | 862,25 | n/a | no history |
| II-150/1 | 173,37 | 724,01 | 1086,40 | n/a | no history |
| II-281/1 | 60,02 | 172,76 | 1093,90 | n/a | no history |
| II-138/1 | 106,36 | 204,60 | 1125,07 | n/a | no history |
| II-252/1 | 261,18 | 796,16 | 1216,87 | II | yes |
| II-274/1 | 237,25 | 592,11 | 1345,67 | n/a | no history |
| II-237/1 | 36,41 | 39,23 | 1388,34 | II | yes |
| II-153/1 | 209,43 | 458,34 | 1399,93 | n/a | no history |

### Group 4: NT-proBNP > 1500 pg/ml

| | Labor | | | NYHA | Heart failure |
|---|---|---|---|---|---|
| Pat.Id. | proSP-B | C-frag. proSP-B | proBNP | class | |
| | ng/ml | ng/ml | pg/ml | | |
| II-083/1 | 156,65 | 286,28 | 1518,29 | II | yes |
| II-108/1 | 48,82 | 117,67 | 1658,79 | II | yes |
| I-55/1 | 91,79 | 634,73 | 1676,53 | n/a | no history |
| II-291/1 | 89,07 | 165,11 | 1701,06 | n/a | no history |
| II-236/1 | 258,70 | 431,21 | 1751,59 | n/a | no history |
| II-096/1 | 143,96 | 293,47 | 1819,22 | n/a | no history |
| II-063/1 | 441,96 | 1010,99 | 2004,89 | n/a | no history |
| II-245/1 | 56,67 | 90,95 | 2159,57 | n/a | no history |
| II-098/1 | 58,56 | 63,88 | 2232,00 | II | yes |
| II-263/1 | 175,65 | 497,83 | 2589,81 | n/a | no history |
| I-04/1 | 131,82 | 403,62 | 2613,03 | III | yes |
| II-066/1 | 511,48 | 812,35 | 2727,45 | II | yes |
| II-091/1 | 40,57 | 64,20 | 2734,00 | II | yes |
| I-53/1 | 80,60 | 127,85 | 2954,92 | II | yes |
| II-051/1 | 70,56 | 208,91 | 3178,20 | n/a | no history |
| I-35/1 | 197,77 | 412,72 | 3629,01 | II | yes |
| II-194/1 | 87,29 | 156,07 | 4181,72 | I | yes |
| II-159/1 | 65,72 | 134,79 | 4579,72 | III | yes |
| II-283/1 | 223,77 | 598,89 | 4675,31 | n/a | no history |
| II-052/1 | 222,49 | 558,41 | 4898,05 | n/a | no history |
| II-282/1 | 68,33 | 125,25 | 5017,19 | III | yes |
| I-11/1 | 340,22 | 926,04 | 5067,19 | n/a | no history |
| II-273/1 | 263,48 | 471,77 | 5131,80 | III | yes |
| II-095/1 | 359,24 | 580,02 | 5481,63 | II | yes |
| II-068/1 | 259,97 | 629,86 | 5629,52 | n/a | no history |
| I-05/1 | 5,60 | 87,03 | 5917,05 | n/a | no history |
| II-279/1 | 508,57 | 2800,55 | 6167,29 | III | yes |
| II-287/1 | 695,41 | 2605,33 | 6173,11 | n/a | no history |
| II-009/1 | 160,77 | 337,28 | 6352,25 | n/a | no history |
| I-28/1 | 111,84 | 217,20 | 6364,04 | n/a | no history |
| II-062/1 | 306,53 | 1525,65 | 8483,27 | III | yes |
| I-59/1 | 453,19 | 785,75 | 8588,84 | n/a | no history |
| II-166/1 | 301,73 | 1097,77 | 8809,51 | n/a | no history |
| I-34/1 | 457,88 | 1038,88 | 9956,92 | n/a | no history |
| II-088/1 | 280,44 | 594,03 | 10064,65 | III | yes |
| II-144/1 | 165,98 | 530,56 | 12707,52 | II | yes |
| II-165/1 | 249,77 | 490,12 | 13933,39 | n/a | no history |
| II-234/1 | 124,47 | 314,83 | 14104,15 | III | yes |
| II-198/1 | 321,41 | 1309,86 | 16588,20 | n/a | no history |
| II-145/1 | 133,63 | 310,39 | 18729,52 | n/a | no history |
| II-227/1 | 308,97 | 966,67 | 33327,16 | II | yes |
| II-251/1 | 296,63 | 980,40 | 35000,00 | II | yes |
| I-06/1 | 135,54 | 207,57 | 106790,14 | III | yes |

As can be seen form the tables, in each patient category alveolar damage as assessed by proSP-B varied significantly. NT-pro BNP values of patients who were previously asymptomatic with respect to their cardiac disease that exceeded 500 pg/ml and, if LVEF was impaired (Heart failure: "yes"), exceeded 1500 pg/ml, must be considered to be related to systemic infection and as a precursor of sepsis. In patients who were previously symptomatic, values taken in a stable state are needed to decide on the impact of systemic infection.

Individual cases are discussed in the following Example 4.

### Example 4: Case studies, Part I

### a) Group 1, NT-proBNP below 125 pg/ml

Patient II 280: no history of heart failure, "asymptomtic", pneumonia, NT-pro BNP levels within normal and no evidence of systemic infection, proSP-B indicates existence of non-cardiac lung damage.

Patient II 064: evidence of pneumonia, NT-pro BNP and pro SP-B within normal no cardiac and no non-cardiac lung damage

### b) Group 2, NT-proBNP 125 pg/ml to 500 pg/ml

Patient II 069: evidence of pneumonia, history of heart failure, NT-pro BNP abnormal, pro SP-B indicated non-cardiac (and cardiac) lung damage

Patient II 163: evidence of pneumonia, no history of heart falure, NT.-pro BNP and pro SP-B slightly abnormal, consistent with predominant cardiac lung damage

### c) Group 3, NT-proBNP 500 to 1500 pg/ml

Patient II 150: pneumonia, nor history of heart failure, NT-pro BNP levels consistent with systemic infection and associated lung injury, non-cardiac lung damage

Patient II 237: pneumonia, history of heart failure, increased NT-pro BNP levels consistent with history of heart failure, minor lung damage consistent with cardiac lung damage, no evidence of non-cardiac lung damage.

### d) Group 4, NT-proBNP larger than 1500 pg/ml

Patient II 051: pneumonia, no history of heart failure, significantly increased NT-pro BNP levels consistent with systemic infection and lung injury, non-cardiac lung damage.

Patient II 088: pneumonia, history of heart failure, NT-pro BNP levels exceeding expected NT-pro BNP levels consistent with pre-existing heart failure, systemic infection and significant non-cardiac lung damage.

### Example 5: Case Studies, Part II

A 52 year old man without a history of heart failure however with signs of fatigue since several months presents with fever and dyspnoe to the emergency room, he has minimal infiltrates on chest x ray, while blood cell counts are within normal, his pro SP-B is 65 ng/ml, he is diagnosed with viral pneumonia and treated symptomatically.

A 46 year old obese patient without a history of heart failure but long lasting dyspnoe on exertion presents with fever to the emergency room, he has questionable infiltrates on chest x ray, his proSP-B is 32 ng/ml, He is diagnosed with upper respiratory tract infection and without evidence of pneumonia and heart failure and it sent home with symptomatic treatment.

A 71 year old woman (no history of heart failure) with fever and dyspnoe to the emergency room, the takes steroids because of underlying osteoarthritis, chest x ray reveals lobular pneumonia, her NT-pro BNP in 1200 pg/ml and pro SP-B is 102 ng/ml., she is diagnosed with suspected bacterial pneumonia and systemic infection and considered to have acute lung injury and early sepsis and immediately put on antibiotics.

A 74 year old male with a history of heart failure and NYHA class III presents with fever and dyspnoe to the emergency room, his NT-pro BNP is 4200 pg/ml and pro SP-B is 165 ng/ml, on chest x ray he lobular pneumonia, he is diagnosed with pneumonia and systemic infection and early sepsis as well as left sided heart failure. He is treated with antibiotics and diuretics for underlying heart failure.

A 68 year old male with a history of heart failure (NHYA class IV) develops fever and presents to the emergency room, on chest X ray there are faint infiltrates, his NT-pro BNP is 995 pg/ml and his pro SP-B is 65 ng/ml. NT-pro BNP and pro SP-B values are considered to be linked to heart failure and a superimposed pulmonary infection appears unlikely. He is diagnosed with upper respiratory tract infection, treated symptomatically and heart failure therapy is optimized. No lung injury was detected.

### Conclusions:

ProSP-B and the c-fragment proSP-B are powerful indicators of injury of alveolar type 2 cells and thus of alveolar injury. Increased pro SP-B levels have been linked to cardiac and non cardiac lung injury. Pneumonia is a disease affecting the lungs caused by bacteria, virus, fungi and protozoa which might occur in predisposed (asympomatic heart failure) individuals and may proceed to systemic infection with consecutive lung injury.

The present invention utilizes proSP- B and NT-pro BNP (which is a marker of cardiac function and heart failure and also of cardiac involvement in systemic infection) to dissect different clinical states associated with increases of NT-pro BNP and pro SP-B. The dissection of these clinical states has impact on treatment.

As shown it was possible to dissect cardiac from non cardiac lung injury in patients without a definite history of heart failure using defined reference values. In another aspect it was possible to identify individuals with pneumonia and systemic infection and suspected lung injury, using appropriate reference values of NT-pro BNP. Thus the present invention allows to differentiate different clinical states associated with pneumonia that have therapeutic consequences especially as these events are detected at an early stage.

## Claims

1. A method for diagnosing non-cardiac lung damage in a subject suffering from pneumonia, said subject having no history of heart failure, comprising the steps of
a) determining the amount of proSP-B in a sample from the subject, and
b) comparing the, thus, determined amount to a reference amount,
wherein the reference amount is derived from a reference subject suffering from pneumonia, but having no history of heart failure, or from a group thereof.

2. The method of claim 1, wherein i) the subject to be tested and ii) the reference subject is asymptomatic with respect heart failure, in particular wherein the subject to be tested and the reference subject are classified as NYHA class I or class II subjects.

3. The method of claims 1 and 2, wherein the reference subject suffers from non-cardiac lung damage, and wherein an essentially identical or increased amount of proSP-B in the sample from the subject to be tested as compared to the reference amount indicates that the subject suffers from non-cardiac lung damage.

4. The method of claims 1 and 2, wherein the reference subject does not suffer from non-cardiac lung damage, and where an essentially identical or decreased amount of proSP-B in the sample from the subject to be tested as compared to the reference amount indicates that the subject does not suffer from non-cardiac lung damage.

5. A method for the diagnosis of acute lung injury in a subject who suffers from pneumonia, comprising the steps of
a) determining the amount of a brain natriuretic peptide in a sample from the subj ect, and
b) comparing the, thus, determined amount to a reference amount, thereby diagnosing acute lung injury,
wherein
i) the reference amount is derived from a reference subject who has no history of heart failure, if the subject to be tested also has no history of heart failure,
ii) the reference amount is derived from a reference subject who suffers from heart failure classified as NYHA class III, if the subject to be tested suffers from heart failure classified as NYHA class III, and/or
iii) the reference amount is derived from a reference subject who suffers from heart failure classified as NYHA class IV, if the subject to be tested suffers from heart failure classified as NYHA class IV.

6. The method of claim 5,
a) wherein the reference subject as defined in i) to iii) is known to suffer from acute lung injury, and wherein an amount of the brain natriuretic peptide in the sample of the subject to be tested which is essentially identical or which is increased as compared to the reference amount, indicates that the subj ect suffers from acute lung injury, and/or
b) wherein the reference subject as defined in i) to iii) is known not to suffer from acute lung injury, and wherein an amount of the brain natriuretic peptide in the sample of the subject to be tested which is essentially identical or which is decreased as compared to the reference amount, indicates that the subject does not suffer from acute lung injury.

7. The method of claims 5 and 6, wherein the brain natriuretic peptide is NT-proBNP.

8. The method of any one of claims 5 to 7 further comprising the step of determining the amount of proSP-B in a sample from the subject and comparing the, thus, determined amount to a reference amount.

9. The method of any one of claims 5 to 8, wherein the subject who has no history of heart failure is apparently healthy with respect to heart failure, or is asymptomatic with respect to heart failure.

10. The method of any one of claims 1 to 9, wherein the sample is blood, serum or plasma.

11. Use of the biomarker proSP-B or a detection agent that specifically binds thereto in a sample of a subject who has no history of heart failure and who suffers from pneumonia for diagnosing non-cardiac lung damage.

12. Use of a brain natriuretic peptide or of a detection agent that specifically binds thereto in a sample of a subject suffering from pneumonia for diagnosing acute lung injury.

13. The use of claim 13, further comprising the use of proSP-B, or of a detection agent which specifically binds thereto in a sample of the subject for diagnosing acute lung injury.

14. A device adapted for carrying out the method of any one of claims 1 to 4, 9 and 10 comprising
a) an analyzing unit comprising a detection agent which specifically binds to proSP-B adapted for determining the amount of proSP-B in a sample of a subject suffering from pneumonia who has no history of heart failure; and
b) an evaluation unit for comparing the determined amount with a reference whereby it can be diagnosed whether the subject suffers from non cardiac lung damage, said unit comprising a database with a reference amount from a reference subject suffering from pneumonia, but having no history of heart failure and a computer-implemented algorithm for carrying out a comparison as defined in claim 3 and/or 4.

15. A kit adapted for carrying out the method of any one of claims 1 to 4, 9 and 10 comprising a detection agent for the biomarker proSP-B, at least one standard for a reference and instructions for carrying out the said method.
